(19) Europäisches Patentamt
European Patent Office
Office européen des brevets

(11) **EP 3 585 334 B1**

(12) **FASCICULE DE BREVET EUROPEEN**

(45) Date de publication et mention
de la délivrance du brevet:
**17.03.2021 Bulletin 2021/11**

(21) Numéro de dépôt: **18709955.1**

(22) Date de dépôt: **23.02.2018**

(51) Int Cl.:
*A61F 9/009* (2006.01)    *A61F 9/008* (2006.01)

(86) Numéro de dépôt international:
**PCT/EP2018/054473**

(87) Numéro de publication internationale:
**WO 2018/154038 (30.08.2018 Gazette 2018/35)**

(54) **INTERFACE DE COUPLAGE ENTRE UNE SOURCE LASER ET UN TISSU A TRAITER**

KOPPELSCHNITTSTELLE ZWISCHEN EINER LASERQUELLE UND EINEM ZU BEHANDELNDEN GEWEBE

COUPLING INTERFACE BETWEEN A LASER SOURCE AND A TISSUE TO BE TREATED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.02.2017 FR 1751469**

(43) Date de publication de la demande:
**01.01.2020 Bulletin 2020/01**

(73) Titulaire: **Keranova**
**42000 Saint Etienne (FR)**

(72) Inventeurs:
• **ROMANO, Fabrizio**
**01700 Beynost (FR)**
• **BERNARD, Aurélien**
**42000 Saint Etienne (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 1 970 034       WO-A1-2017/055252**
**US-A1- 2011 319 873**

**Description**

**DOMAINE TECHNIQUE**

La présente invention concerne le domaine technique des opérations chirurgicales réalisées au laser, et plus particulièrement celui de la chirurgie ophtalmologique pour notamment des applications de découpe de cornées, ou de cristallins.

L'invention concerne une interface d'adaptation permettant le couplage d'une source laser au tissu humain ou animal devant être traité, tel qu'une cornée, ou un cristallin.

*Par « source laser »,* on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

**ART ANTERIEUR**

Il est connu de l'état de la technique de réaliser des opérations chirurgicales de l'œil au moyen d'une source laser, telles que des opérations de découpe de cornées ou de cristallins.

Une source laser est un instrument apte à réaliser une découpe du tissu cornéen par exemple, en focalisant un faisceau L.A.S.E.R. dans le stroma de la cornée, et en réalisant une succession de petites bulles de cavitation adjacentes, qui forment ensuite une ligne de découpe.

Plus précisément, lors de la focalisation du faisceau L.A.S.E.R. dans la cornée, un plasma est généré par ionisation non-linéaire lorsque l'intensité de la source laser dépasse une valeur seuil, nommée seuil de claquage optique. Une bulle de cavitation se forme alors, engendrant une disruption très localisée des tissus environnants. Ainsi, le volume réellement ablaté par la source laser est très faible comparativement à la zone disruptée.

Pour permettre le couplage de la source laser à l'œil d'un patient, il est connu d'utiliser une interface d'adaptation disposée entre l'œil du patient et la source laser.

Une telle interface de couplage permet de maintenir le globe oculaire dans une position stable et constante, centrée et à une distance connue de la source laser, en évitant tout mouvement, pendant la durée d'un traitement.

*1. Interface de couplage connue de l'art antérieur*

*1.1.Premier exemple d'interface de couplage existante*

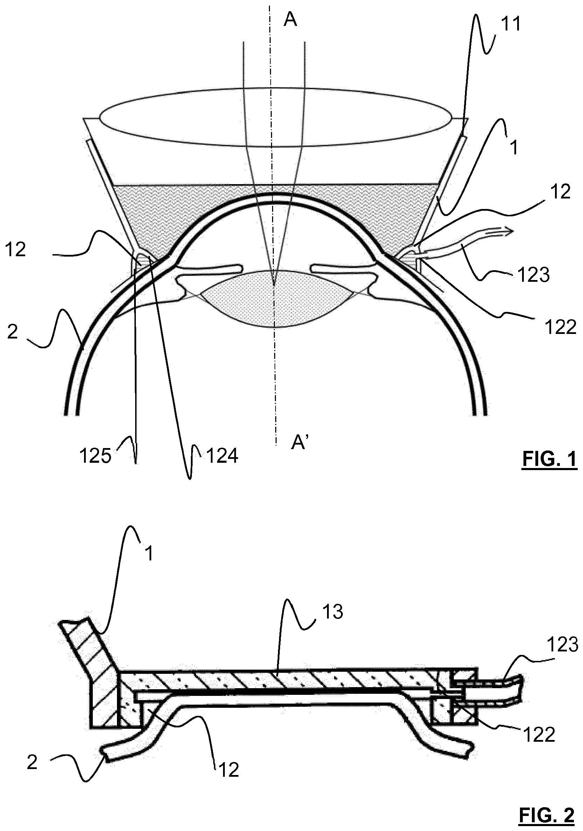

En référence à la figure 1, on a illustré un premier exemple d'interface de couplage de l'art antérieur. L'interface de couplage comprend un anneau incluant un tronc de cône 1 d'axe A-A'. Le tronc de cône 1 est ouvert à ses deux extrémités. La grande base 11 du tronc de cône 1 est destinée à recevoir l'extrémité de la source laser. La petite base du tronc de cône 1 est destinée à venir en contact avec l'œil 2. L'interface de couplage comprend également :

- une gorge annulaire 12 de circulation de gaz s'étendant à la périphérie de la petite base, et
- un membre d'accès tubulaire 122 s'étendant radialement vers l'extérieur et permettant le raccordement de la gorge annulaire 12 à un dispositif d'aspiration externe (non représenté) par l'intermédiaire d'une tubulure de connexion 123.

La gorge annulaire 12 présente une forme de U en section définie par deux bordures circulaires 124, 125 destinées à être plaquées contre l'œil 2 du patient. Le dispositif d'aspiration est connu en soit par l'homme du métier et permet de créer une dépression dans la gorge annulaire 12 une fois les bordures circulaires 124, 125 appliquées sur l'œil 2. La génération d'une dépression dans la gorge annulaire 12 permet de solidariser l'interface de couplage sur l'œil 2 par effet ventouse pendant toute la durée du traitement.

*1.2.Principe de fonctionnement du premier exemple d'interface de couplage existante*

Le principe de fonctionnement d'une telle interface de couplage est le suivant.

Dans un premier temps, le praticien dispose l'anneau de l'interface de couplage sur l'œil 2.

Une fois l'anneau correctement positionné et centré, le dispositif d'aspiration est activé pour générer une dépression dans l'espace défini entre la gorge annulaire 12 et l'œil 2 afin de solidariser l'interface de couplage sur l'œil 2 par ventousage.

Lorsque l'anneau est solidaire de l'œil 2, l'anneau est rempli d'un liquide ayant un indice de réfraction proche de celui de la cornée jusqu'à immersion de la cornée. Le liquide immergeant ainsi la cornée, a l'avantage de simplifier la trajectoire des faisceaux L.A.S.E.R. à travers la cornée, ceux-ci traversant une surface plane, plutôt qu'une surface incurvée, la cornée du patient, qui de plus est souvent affectée de défauts optiques mineurs (astigmatisme) ajoutant des possibles aberrations sur la trajectoire du faisceau L.A.S.E.R..

La source laser est ensuite placée au droit de l'interface de couplage, la partie distale de la source laser étant insérée et verrouillée sur l'interface de couplage, de manière à ce que l'œil 2 et l'axe optique du laser soient alignés et maintenus solidairement pendant la durée du traitement.

### 1.3. Inconvénients du premier exemple d'interface de couplage existante

Une telle interface de couplage présente de nombreux inconvénients.

### 1.3.1. Pression exercée sur l'interface pour assurer l'étanchéité

En particulier, l'efficacité du ventousage de l'interface de couplage sur l'œil 2 dépend de la qualité de l'étanchéité entre les bordures circulaires 124, 125 et l'œil 2.

Un contact parfait, doit donc être obtenu sur toute la circonférence de la ligne de contact de chaque bordure circulaire 124, 125.

Ceci n'est pas toujours simple, les yeux des patients étant tous différents, la surface du globe ne suit pas forcément la pente entre chaque bordure circulaire 124, 125.

Pour pallier cette inhomogénéité entre les yeux des patients, il est donc nécessaire d'appuyer fortement l'anneau de l'interface de couplage pour obtenir une bonne étanchéité entre la gorge 12 et l'œil 2, ce qui est préjudiciable au confort et à la sécurité du patient. En effet, la force exercée sur l'anneau de l'interface de couplage pour obtenir une étanchéité satisfaisante tend à faire pénétrer les bordures circulaires 124, 125 dans la conjonctive.

La force à appliquer pour obtenir l'étanchéité est parfois si importante que l'inconfort du patient peut tendre vers la douleur. Par ailleurs, cette pression, même si de courte durée, provoque immédiatement l'augmentation de la pression intraoculaire du patient, ce qui chez certains patients à risque (forts myopes, antécédents de décollement de rétine ou glaucome), peut se traduire par des effets secondaires graves.

### 1.3.2. Temps nécessaire à l'obtention d'une dépression

Par ailleurs, comme décrit précédemment, le ventousage de l'interface de couplage sur l'œil 2 est obtenu en utilisant un dispositif d'aspiration générant une dépression dans l'espace délimité entre la gorge 12 et l'œil 2.

Un gaz étant compressible et extensible, la force d'aspiration dépend du volume de gaz compris dans cet espace étanche. Plus ce volume est important, plus la force d'aspiration augmente lentement.

Certaines interfaces de couplage traditionnelles comprennent des tubulures entièrement remplies de gaz, ce qui allonge le temps nécessaire à l'obtention d'un ventousage satisfaisant.

### 1.3.3. Intensité de la dépression

Egalement, l'intensité de la dépression doit être importante pour assurer le maintien en position de l'interface de couplage.

En effet, pour résister aux micromouvements oculaires et à la tendance au dé-ventousage, la force d'aspiration doit être importante.

Or il est connu que la Force F d'aspiration est fonction de la Dépression P et de la Surface S sur laquelle est appliquée cette dépression, soit :

$$F = P \times S.$$

Dans les interfaces de couplage traditionnelles, la surface sur laquelle est appliquée la dépression étant faible (i.e. limitée à la surface de l'œil entre les deux bordures circulaires), il est nécessaire d'appliquer une dépression de valeur importante pour obtenir une force d'aspiration F permettant de résister aux micromouvements oculaires et à la tendance au dé-ventousage.

L'importance de la dépression appliquée entraîne parfois l'apparition d'hémorragies conjonctivales (effusion de globules rouges liée à l'aspiration sous la conjonctive). Même si des ecchymoses superficielles sont généralement très limitées, et n'ont aucune conséquence sur la vision, il est préférable de limiter leur apparition.

### 1.3.4. *Manipulation*

Lorsque le ventousage est obtenu, l'anneau de succion devient solidaire du globe oculaire et le cône renversé à ciel ouvert délimite un volume V pouvant être rempli d'un liquide à l'indice de réfraction proche de celui de la cornée, comme de l'eau ou du sérum physiologique.

Cette étape d'immersion est habituellement réalisée manuellement en déversant dans le cône une quantité de liquide suffisante pour affleurer en haut du cône.

Avec certaines interfaces de couplage traditionnelles, il est nécessaire de réaliser des manipulations :

- saisir un flacon,
- dévisser le capuchon,
- se mettre à la verticale et en visuel pour remplir le cône,
- déverser suffisamment de liquide dans le cône.

Ces manipulations sont parfois longues et imprécises, allongeant le temps de ventousage qui doit être réduit au minimum.

### 1.3.5. *Coût*

Certaines interfaces de couplage traditionnelles, fonctionnent avec des équipements chirurgicaux spécifiques. Ceux-ci ont dû intégrer un système de ventousage, avec pompe, tubulures et capteur de dépression. Chaque système étant spécifique, il est nécessaire d'acheter tous ces accessoires. La présente invention évite de faire cette dépense inutile. En effet, cette interface patient fonctionne avec des accessoires déjà présents au bloc opératoire, et notamment l'appareil de phacoémulsification. Celui-ci est équipé d'une pompe d'aspiration avec capteur de dépression et d'une ligne d'infusion, et dispose déjà des tubulures adéquates. Nos essais expérimentaux démontrent que la nouvelle interface de couplage objet de cette invention, fonctionne parfaitement avec un appareil de phacoémulsification, ce qui génère une économie substantielle pour le client.

De plus si l'appareil de phacoémulsification, équipé de son système de pompe et de capteurs est intégré à l'équipement laser, cela simplifie grandement la configuration au bloc opératoire, une seule machine permettant de réaliser toute la procédure chirurgicale avec un seul consommable et un seul système d'aspiration/irrigation qui devient multifonction, assurant dans un premier temps la réalisation du ventousage de l'interface patient et dans un deuxième temps la réalisation de la fin de l'intervention avec l'irrigation/aspiration des fragments cristalliniens et des masses corticales cristalliniennes.

### 1.2. *Deuxième exemple d'interface de couplage existante*

En référence à la figure 2, on a illustré un deuxième exemple d'interface de couplage de l'art antérieur. Ce deuxième exemple d'interface de couplage diffère du premier exemple en ce que l'interface comprend une lame 13 transparente au rayonnement laser disposée à proximité de la petite base du tronc de cône 1 destinée à venir en contact avec l'œil 2.

Cette lame 13 permet d'aplanir la cornée 2 du patient. Un tel aplanissement de la cornée est nécessaire lorsque le l'interface de couplage ne comprend pas de liquide pour diminuer le dioptre cornéen. Toutefois, l'aplanissement de la face antérieure de la cornée (tel que représenté à la figure 2) induit l'apparition de plis sur la face postérieure de la cornée, ces plis induisant une dégradation de la qualité du traitement par laser. Un tel inconvénient lié au plissement de la face postérieure de la cornée est notamment décrit dans l'article intitulé « Optical patient interface in femtosecond laser-assisted cataract surgery: Contact corneal applanation versus liquid immersion », de Jonathan H. Talamo, MD, Philip Gooding, MS, David Angeley, MS, William W. Culbertson, MD, Georg Schuele, PhD, Daniel Andersen, BS, George Marcellino, PhD, Emma Essock-Burns, PhD, Juan Batlle, MD, Rafael Feliz, MD, Neil J. Friedman, MD, Daniel Palanker, PhD.

### 1.3. *Autres interfaces de couplage existantes*

Le document EP 1 970 034 décrit anneau-ventouse de chirurgie ophtalmologique, comprenant :

- une première zone ventouse conçue pour appliquer l'anneau-ventouse sur l'œil, et
- une deuxième zone ventouse conçue pour aspirer un élément fonctionnel.

L'élément fonctionnel et/ou l'anneau-ventouse présentent éventuellement des moyens de mesure. L'élément fonctionnel peut être de type réceptacle de manière à contenir un liquide qui, en mode d'utilisation, se situe entre la cornée de l'œil et une lentille.

Le document US 2011/319873 décrit une interface patient de système ophtalmique comprenant :

- un module de fixation pouvant être fixé au système ophtalmique, et
- un module de contact, configuré pour recevoir une substance viscoélastique entre l'interface patient et l'œil soumis à l'intervention.

La substance viscoélastique peut comprendre un fluide, un liquide, un gel, une crème, une larme artificielle, un film, une matière élastique ou une matière visqueuse. L'interface patient peut également comprendre des orifices d'entrée, des orifices de sortie et un système d'aspiration.

*2. But de l'invention*

Un but de la présente invention est de proposer une interface de couplage permettant de pallier au moins l'un des inconvénients précités.

**EXPOSE DE L'INVENTION**

A cet effet l'invention propose une interface de couplage entre une source laser et un tissu à traiter, remarquable en ce que l'interface de couplage comprend

- un anneau incluant :

  • une paroi latérale,
  • une extrémité proximale destinée à venir en contact avec le tissu à traiter, et
  • une extrémité distale destinée à recevoir l'extrémité de la source laser

- une fenêtre transparente à un faisceau laser généré par la source laser, ladite fenêtre étant montée de manière étanche sur l'anneau de sorte à fermer l'extrémité distale de l'anneau
- un canal d'aspiration traversant s'étendant entre les faces interne et externe de la paroi latérale, le canal d'aspiration étant destiné à être connecté à un module d'aspiration pour permettre la génération d'une dépression dans un espace intérieur défini entre la fenêtre et la paroi latérale,
- un canal d'irrigation traversant s'étendant entre les faces interne et externe de la paroi latérale, le canal d'irrigation étant destiné à être connecté à un module d'irrigation pour permettre l'infusion de liquide dans l'espace intérieur.

Ainsi, la présente invention propose la fourniture d'une interface de couplage - notamment utilisable dans le cadre d'une chirurgie ophtalmique laser - à ventousage rapide et indolore et infusion simultanée et automatique de liquide, cette interface de couplage étant compatible avec l'utilisation de modules d'irrigation et d'aspiration, qui peuvent être intégrés dans un appareil couramment présent en bloc opératoire ophtalmique : le phacoémulsificateur. Ainsi l'interface de couplage peut être utilisée dans un dispositif incluant une source laser et un phacoémulsificateur, ce dispositif comprenant un unique module d'irrigation et d'aspiration (pompe + capteur de dépression) utilisé à la fois pour le traitement laser et le traitement de phacoémulsification. Ceci permet de limiter les coûts en intégrant dans un même dispositif des éléments communs à deux modules spécifiques (i.e. la source laser d'une part et le phacoémulsificateur) habituellement séparés au bloc opératoire.

Bien entendu, la présente invention est également compatible avec l'utilisation de modules d'irrigation et d'aspiration non intégrés, comprenant par exemple un (ou plusieurs) récipient(s) tel(s) qu'une seringue, une (ou plusieurs) tubulure(s), et une (ou plusieurs) pompe telle(s) qu'un pousse seringue électrique.

La présence d'un canal d'irrigation débouchant dans l'espace défini entre la fenêtre et la paroi latérale de l'anneau permet de s'affranchir de la nécessité d'aplanissement de la cornée pour réduire le dioptre cornéen. Il est ainsi possible de déporter la fenêtre à proximité de l'extrémité distale de l'anneau afin d'éviter tout contact entre l'œil du patient et la fenêtre. Des aspects préférés mais non limitatifs de l'interface de couplage sont les suivants :

- l'interface de couplage peut comprendre en outre un piège à bulles s'étendant autour de la fenêtre ;
- le piège à bulles peut consister en une rainure interne circulaire s'étendant entre la paroi latérale et la fenêtre ;
- le canal d'aspiration peut s'étendre dans un plan contenant la fenêtre ;
- la fenêtre peut être recouverte d'une couche de matériau hydrophile ;
- la fenêtre peut avoir une géométrie non plane à faces parallèles, présentant par exemple une concavité permettant une meilleure évacuation des bulles vers le piège à bulles ;
- la fenêtre peut avoir une géométrie non plane à faces non parallèles, présentant par exemple une biconvexité, conférant ainsi à la fenêtre un rôle optique ;
- l'anneau comporte un col évasé de forme générale tronconique incurvée vers l'extérieur au niveau de son extrémité proximale, le col évasé étant apte à être appliqué sur la surface externe du tissu à traiter ;
- la fenêtre peut être fixée à l'anneau par collage ;
- le diamètre intérieur du canal d'irrigation peut être inférieur au diamètre intérieur du canal d'aspiration.

La divulgation concerne également un procédé d'installation d'une interface de couplage entre une source laser et un tissu à traiter, l'interface de couplage comprenant un anneau incluant :

- une paroi latérale,
- une extrémité proximale destinée à venir en contact avec le tissu à traiter,
- une extrémité distale destinée à recevoir l'extrémité de la source laser et
- une fenêtre transparente à un faisceau laser généré par la source laser montée de manière étanche sur l'extrémité distale de l'anneau,

le procédé comprenant les étapes suivantes :

- Positionnement de l'anneau sur le tissu à traiter
- Activation simultanée d'un dispositif d'aspiration et d'un dispositif d'irrigation de liquide :

  ○ l'activation du dispositif d'aspiration permettant de créer une dépression dans un espace intérieur défini entre la fenêtre, la paroi latérale et le tissu à traiter,
  ○ l'activation du dispositif d'irrigation permettant de remplir l'espace intérieur en liquide,

- Désactivation du dispositif d'irrigation lorsque le liquide est en contact avec la fenêtre.
- Désactivation de la pompe d'aspiration dès que le seuil de dépression désiré est atteint
- Maintien de la dépression constante, pendant toute la durée de la procédure.

Une fois l'intervention terminée, l'interface de couplage est désolidarisée du tissu à traiter en rétablissant la pression atmosphérique dans l'espace intérieur défini entre la fenêtre, la paroi latérale et le tissu traité.

**BREVE DESCRIPTION DES DESSINS**

D'autres caractéristiques et avantages de l'invention ressortiront clairement de la description qui en est réalisée ci-après, à titre indicatif et nullement limitatif, en référence aux figures annexées, dans lesquelles :

- la figure 1, 2 et 4B sont des représentations schématiques d'une interface de couplage de l'art antérieur ;
- les figures 3, 4A, 5 et 6 sont des représentations schématiques d'une interface de couplage selon l'invention,
- la figure 7 illustre un procédé d'installation de l'interface de couplage selon l'invention.

**EXPOSE DETAILLE DE L'INVENTION**

On va maintenant décrire l'interface de couplage selon l'invention en référence aux figures. Dans ces différentes figures, les éléments équivalents sont désignés par la même référence numérique.

L'interface de couplage est destinée à être disposé entre une source laser et une cible à traiter 2. La cible 2 est par exemple un tissu humain ou animal à traiter tel qu'un globe oculaire et plus précisément une cornée ou un cristallin.

Dans la suite de la description, l'invention sera décrite, à titre d'exemple, pour le traitement d'une cornée d'un œil humain ou animal. Néanmoins, il est bien évident pour l'homme du métier que l'interface de couplage selon l'invention peut être utilisée dans d'autres applications.

*1. Description générale de l'interface de couplage*

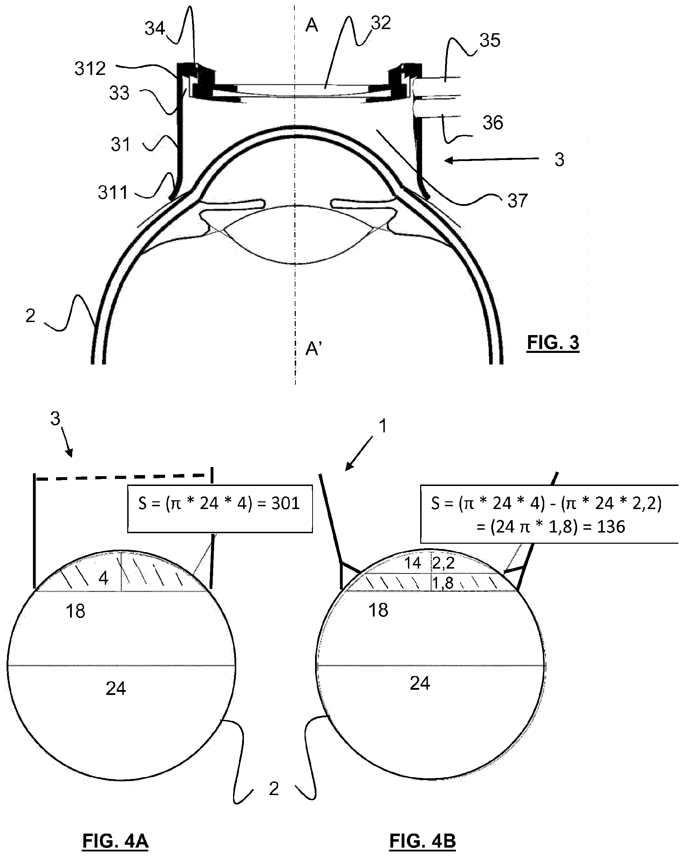

En référence à la figure 3, l'interface de couplage comprend un anneau 3 incluant :

- une paroi latérale 31 présentant une forme générale cylindrique ou conique
- une extrémité proximale 311 destinée à venir en contact avec un œil 2 à traiter, et

- une extrémité distale 312 destinée à recevoir l'extrémité de la source laser.

L'interface de couplage est remarquable en ce qu'elle comprend en outre une fenêtre 32 transparente au faisceau L.A.S.E.R. montée de manière étanche sur l'extrémité distale 312 de l'anneau 3 de sorte à fermer l'extrémité distale 312 de l'anneau 3. L'anneau 3 n'est donc ouvert qu'à son extrémité proximale 311 destinée à venir en contact avec l'œil 2 du patient.

La fixation de l'interface de couplage sur l'œil 2 du patient peut alors être réalisée en générant une dépression dans l'espace intérieur 37 défini entre :

- la paroi latérale 31 de l'anneau 3,
- la fenêtre transparente 32, et
- l'œil 2 du patient.

Ainsi et contrairement aux interfaces de couplage existantes, la fixation de l'interface de couplage sur l'œil 2 par la génération d'une dépression, ne nécessite pas d'avoir un appui parfait entre deux bordures circulaires définissant une gorge annulaire.

En raison de cette nouvelle conception, le niveau de vide (i.e. l'intensité de la dépression) nécessaire pour assurer le maintien en position de l'interface de couplage est considérablement réduit puisque la force d'aspiration s'applique cette fois-ci à toute la surface du globe recouverte par l'anneau 3.

En effet, comme illustré à la figure 4B qui est une représentation schématique de l'interface de couplage traditionnelle de la figure 1, pour obtenir une force d'aspiration de par exemple 3 Newtons, il faut appliquer une dépression de :

$$P = F / S,$$

soit

$$P = 3 / 136.10^{-6},$$

soit

$$P = 22058 \text{ Pa} = 0.22 \text{ bar} = 165 \text{ mmHg.}$$

Avec l'interface de couplage illustrée à la figure 3, pour une même force d'aspiration de 3 Newtons, la dépression nécessaire est uniquement de :

$$P = F / S,$$

soit

$$P = 3 / 301.10^{-6},$$

soit

$$P = 9966 \text{ Pa} = 0.099 \text{ bar} = 74 \text{ mmHg (cf. figure 4A).}$$

Ainsi avec l'interface de couplage illustrée à la figure 3, la fixation de l'anneau 3 sur l'œil 2 est obtenue en générant une dépression très inférieure à la dépression nécessaire pour maintenir les interfaces de couplage traditionnelles.

*2. Description détaillée de l'interface de couplage*

*2.1.Anneau*

Dans le mode de réalisation illustré à la figure 3, l'anneau 3 comporte une paroi latérale 31 présentant une forme générale cylindrique. Toutefois, il est bien évident pour l'homme du métier que l'anneau 3 peut présenter d'autres formes à symétrie de révolution autour d'un axe A-A', telle qu'une forme tronconique.

L'extrémité proximale 311 de l'anneau 3 comporte un col évasé de forme générale tronconique incurvée vers l'extérieur, et apte à être appliqué sur la surface externe de l'œil 2 de manière non traumatisante. Ce col évasé présente un profil concave de rayon de courbure sensiblement égal au rayon de courbure de l'œil 2 permettant un appui tangentiel.

Le fait que l'anneau comprenne un unique col évasé (plutôt que deux bordures circulaires dans les interfaces de couplage traditionnelles) permet de faciliter l'obtention d'un contact parfait sur toute la circonférence de la ligne de contact entre l'œil 2 et l'anneau 3.

L'anneau 3 comprend une couronne annulaire 34 s'étendant radialement vers l'intérieur au niveau de son extrémité distale 312. La couronne annulaire 34 comprend un décrochement sur sa face latérale formant berceau pour la réception de la fenêtre transparente 32. Le décrochement permet de faciliter la mise en place de la fenêtre transparente 32. Toute autre méthode permettant la fixation de manière étanche d'une fenêtre transparente sur la partie distale, par soudure, collage ou vissage par exemple, est bien entendu envisageable.

La couronne annulaire 34 comprend également une rainure circulaire 33 s'étendant entre la paroi latérale 31 et la fenêtre transparente 32. Cette rainure circulaire 33 forme un piège à bulles d'air. En effet, des bulles d'air sont susceptibles de se former lors du remplissage de l'interface de couplage avec le liquide ayant un indice de réfraction proche de celui de la cornée. Ces bulles d'air peuvent nuire à l'efficacité du traitement, notamment si celles-ci sont positionnées sur le chemin de propagation du faisceau L.A.S.E.R. émis par la source laser. La présence d'un piège à bulles d'air permet de retenir les bulles d'air générées lors du remplissage de l'interface de couplage en dehors du chemin de propagation du faisceau L.A.S.E.R. Ceci permet de garantir au praticien une bonne efficacité de traitement. Ceci peut également être facilité par une géométrie non plane de la fenêtre transparente, par exemple avec une convexité vers l'œil 2, tout en gardant des faces parallèles, de manière à améliorer l'acheminement des bulles de gaz vers cette rainure.

*2.2.Fenêtre*

En référence à la figure 3, la fenêtre 32 transparente au faisceau généré par la source laser présente forme d'un disque. Bien entendu, la fenêtre peut présenter d'autres formes (carrée, rectangulaire, elliptique) en fonction de l'application visée.

La fenêtre 32 peut être conçue en différents matériaux tels que du verre ou du plastique (Polycarbonate, Poly(méthacrylate de méthyle), etc.).

Dans le mode de réalisation illustré à la figure 3, la fenêtre 32 et l'anneau 3 sont constitués en deux pièces distinctes. Néanmoins, dans certains modes de réalisation, la fenêtre 32 et l'anneau 3 peuvent être constitués en une seule pièce (monobloc). Ceci permet de limiter les risques de fuite à la jonction entre l'anneau 3 et la fenêtre 32.

Lorsque la fenêtre 32 et l'anneau 3 sont deux pièces distinctes de l'interface de couplage, la fenêtre peut être fixée à l'anneau 3 par collage, soudage ou toute autre technique permettant la fixation de manière étanche de la fenêtre 32 sur l'anneau 3.

La fenêtre 32 peut être recouverte d'une couche de matériau hydrophile. Ceci permet de limiter l'adhérence de bulles d'air sur la fenêtre 32.

La fenêtre 32 peut être traitée antireflet ou présenter tout autre type de traitement optique afin d'améliorer la transmission du faisceau L.A.S.E.R. en fonction de sa longueur d'onde.

*2.3. Canaux*

L'interface de couplage comprend également deux canaux tubulaires traversants ménagés dans la paroi latérale 31 de l'anneau 3.

Chaque canal traversant 35, 36 débouche sur la face interne de la paroi latérale 31 de l'anneau, et s'étend radialement vers l'extérieur perpendiculairement à l'axe A-A'.

Chaque canal permet le raccordement d'un dispositif distant à l'interface de couplage par l'intermédiaire d'une tubulure respective :

- Un premier canal 36 (dit « *canal d'irrigation* ») permet le raccordement de l'interface de couplage à un dispositif d'irrigation pour l'infusion de liquide dans l'espace intérieur 37,

- Un deuxième canal 35 (dit « canal d'aspiration ») permet le raccordement de l'interface de couplage à un dispositif d'aspiration pour la génération d'une dépression dans l'espace intérieur 37.

De préférence, le canal d'irrigation 36 s'étend sous le plan contenant la fenêtre 32.

Le canal d'aspiration 35 débouche préférentiellement sur la face interne de la paroi latérale 31 au niveau de la rainure circulaire 33, c'est-à-dire au niveau du plan contenant la fenêtre 32. Ceci permet de garantir que le liquide vienne en contact avec la fenêtre 32 lors du remplissage de l'espace intérieur 37.

Dans le mode de réalisation illustré à la figure 3, les premier et deuxième canaux 35, 36 sont disposés l'un au-dessus de l'autre de sorte que les tubulures - connectées aux canaux d'une part et aux dispositifs d'irrigation et d'aspiration d'autre part - soient situées d'un seul et même côté de l'interface de couplage, ce côté correspondant au côté temporal de la tête du patient afin que les tubulures n'entrent pas en contact avec l'arête nasale.

Avantageusement, le canal d'irrigation 35 peut présenter un diamètre inférieur au diamètre du canal d'aspiration 36. Les dispositifs d'irrigation et d'aspiration étant destinés à être activés simultanément, cette différence de diamètre permet de maintenir une légère dépression lors du remplissage de l'espace intérieur 37 en liquide, et ainsi d'assurer l'étanchéité de l'interface de couplage lors de cette étape.

Par exemple, dans le mode de réalisation illustré à la figure 3, le diamètre du canal d'irrigation 35 est choisi égal à 0.5mm, et le diamètre du canal d'aspiration 36 est choisi égal à 1mm.

### 3. Principe de fonctionnement

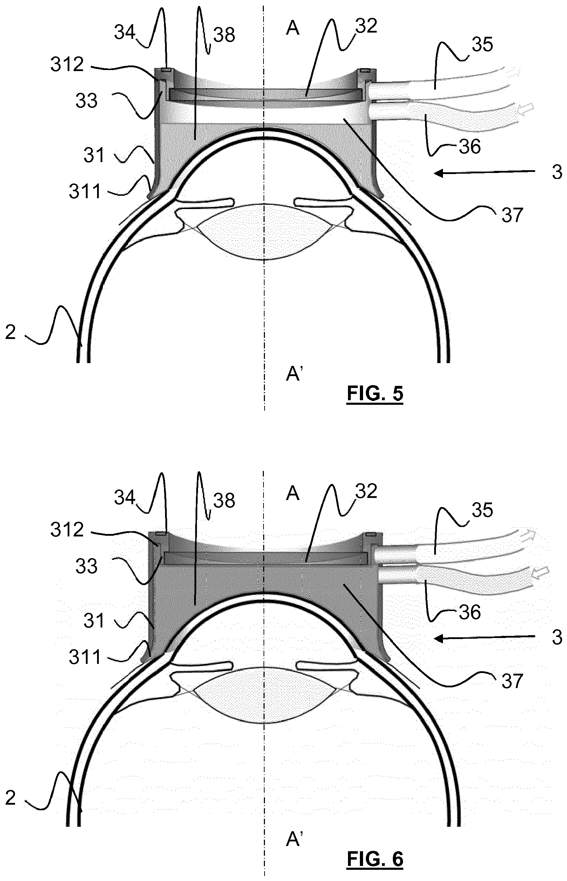

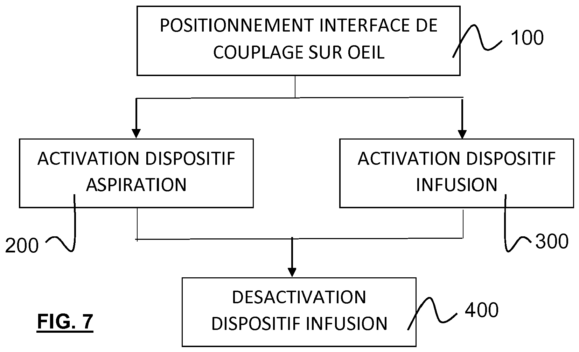

On va maintenant décrire plus en détail le principe de fonctionnement de l'interface de couplage selon l'invention en référence au dispositif de couplage représenté aux figures 3, 5 et 6, et au procédé illustré à la figure 7. On suppose que les premier et deuxième canaux 35, 36 ont été préalablement raccordés au dispositif d'aspiration et d'irrigation (non représentés) par l'intermédiaire de tubulures.

Dans une première étape 100, le praticien positionne l'anneau 3 de l'interface de couplage sur l'œil 2, et centre celui-ci préalablement à la mise en œuvre du traitement par laser.

Dans une deuxième étape 200-300, le praticien active simultanément le dispositif d'aspiration et le dispositif d'irrigation de liquide :

- l'activation du dispositif d'aspiration permet de créer une dépression dans l'espace intérieur 37 qui exerce le ventousage, tandis que
- l'activation du dispositif d'irrigation permet le remplissage de l'espace intérieur 37 en liquide nécessaire pour recouvrir le dôme cornéen et diminuer le dioptre cornéen.

Comme indiqué précédemment, le fait que le diamètre du canal d'irrigation 35 soit astucieusement choisi de taille inférieure (typiquement 0.5mm) par rapport au diamètre du canal d'aspiration 36 (typiquement 1mm) permet de maintenir une légère dépression lors de l'infusion de liquide, et ainsi d'assurer l'étanchéité de l'interface de couplage lors de cette deuxième étape.

L'espace intérieur clos 37 défini entre la cornée 2, la paroi latérale 31 et la fenêtre 32 se remplit en 1 à 2 secondes et simultanément l'air est aspiré, créant un vide suffisant pour le ventousage en 2 à 3 secondes.

Le remplissage est ainsi complet très vite et sans aucune manipulation, et il ne reste pas d'air entre la fenêtre de fermeture 32 et la cornée 2, l'air résiduel étant confiné dans le piège à bulles circulaire 33, dans lequel aboutit le canal d'aspiration 35.

De plus le circuit d'aspiration contient un volume de gaz moins important que les interfaces de couplage traditionnelles, puisqu'une partie de ce gaz est remplacée par du liquide non compressible simultanément à l'aspiration, ce qui augmente la rapidité d'obtention d'un maintien ferme par le ventousage.

Une fois le liquide en contact avec la fenêtre, le praticien désactive le dispositif d'irrigation, tout en maintenant le dispositif d'aspiration activé (étape 400).

L'extrémité de la source laser peut ensuite être fixée à l'interface de couplage, sans qu'il y ait contact entre l'extrémité de la source laser et le liquide 38.

### 4. Conclusions

Les interventions chirurgicales effectuées en ophtalmologie et ayant recours à une source laser (notamment femtoseconde), utilisent habituellement un système de maintien du globe oculaire, qui doit être actif pendant toute la durée d'exposition du patient au faisceau laser.

En effet, le risque serait qu'en cas de mouvement inopiné et non maîtrisé du globe oculaire, le faisceau atteigne des zones non supposées être atteintes et génère des lésions plus ou moins graves des structures intraoculaires.

Le maintien du globe avec un dispositif à la géométrie connue, est aussi une méthode permettant de connaître précisément la position dans l'espace du globe afin de diriger précisément le faisceau laser sur sa cible.

Le maintien du globe est donc une étape primordiale et les dispositifs utilisés, appelés interfaces de couplage, ont une importance capitale.

Toutefois, le maintien du globe oculaire, implique un contact entre un corps étranger et la surface du globe, ainsi que l'application d'une force s'opposant aux mouvements. Cette position ne peut être maintenue longtemps et il est communément admis, que la durée pendant laquelle l'œil du patient reste ventousé à une interface de couplage ne doit pas excéder deux à trois minutes. Au-delà, l'inconfort pour le patient et les risques provoqués par la surpression sur le globe deviennent inacceptables.

Ce temps limité doit être consacré en grande partie à l'exposition au faisceau laser, qui réalise quelques étapes clés de l'intervention chirurgicale. Donc plus le temps consacré à la mise en place de l'interface de couplage est long, plus le temps d'exposition au laser est court.

Il est donc primordial de simplifier ce geste au maximum, afin de réduire le temps, mais aussi d'augmenter le confort du patient et de réduire les risques.

Enfin, la possibilité d'utiliser moins de matériel consommable, et donc de faire des économies considérables, permettra à ce dispositif d'être accessible au plus grand nombre.

La nouvelle interface de couplage décrite précédemment, permet d'apporter tous ces avantages.

Le lecteur aura compris que de nombreuses modifications peuvent être apportées à l'invention décrite précédemment sans sortir matériellement des nouveaux enseignements et des avantages décrits ici.

Par exemple dans la description qui précède, l'interface de couplage était découplée de l'extrémité de la source laser, la procédure de traitement du patient comprenant une étape de mise en place de l'extrémité de la source laser sur l'interface. En variante, l'interface de couplage peut être solidaire de l'extrémité de la source laser, l'étape de centrage de l'interface sur l'œil du patient étant dans ce cas réalisé de manière automatique en utilisant des moyens connus de l'homme du métier.

Par conséquent, toutes les modifications de ce type sont destinées à être incorporées à l'intérieur de la portée des revendications jointes.

## Revendications

1. Interface de couplage entre une source laser et un tissu à traiter (2), **caractérisé en ce que** l'interface de couplage comprend :

    - un anneau (3) incluant :

        • une paroi latérale (31),
        • une extrémité proximale (311) destinée à venir en contact avec le tissu (2) à traiter, et
        • une extrémité distale (312) destinée à recevoir l'extrémité de la source laser,

    - une fenêtre (32) transparente à un faisceau laser généré par la source laser, ladite fenêtre étant montée de manière étanche sur l'anneau (3) de sorte à fermer l'extrémité distale (312) de l'anneau (3),
    - un canal d'aspiration traversant (35) s'étendant entre les faces interne et externe de la paroi latérale (31), le canal d'aspiration (35) étant destiné à être connecté à un module d'aspiration pour permettre la génération d'une dépression dans un espace intérieur (37) défini entre la fenêtre (32) et la paroi latérale (31),
    - un canal d'irrigation traversant (36) s'étendant entre les faces interne et externe de la paroi latérale (31), le canal d'irrigation (36) étant destiné à être connecté à un module d'irrigation pour permettre l'infusion de liquide (38) dans l'espace intérieur (37).

2. Interface de couplage selon la revendication 1, laquelle comprend en outre un piège à bulles s'étendant autour de la fenêtre (32).

3. Interface de couplage selon la revendication 2, dans laquelle le piège à bulles consiste en une rainure interne circulaire (33) s'étendant entre la paroi latérale (31) et la fenêtre (32).

4. Interface de couplage selon l'une quelconque des revendications précédentes, dans laquelle le canal d'aspiration (35) s'étend dans un plan contenant la fenêtre (32).

5. Interface de couplage selon l'une quelconque des revendications précédentes, dans laquelle la fenêtre (32) est recouverte d'une couche de matériau hydrophile.

6.  Interface de couplage selon l'une quelconque des revendications précédentes, dans laquelle la fenêtre (32) a une géométrie non plane à faces parallèles, présentant par exemple une concavité permettant une meilleure évacuation des bulles vers le piège à bulles (312)

7.  Interface de couplage selon l'une quelconque des revendications précédentes, dans laquelle la fenêtre (32) a une géométrie non plane à faces non parallèles, présentant par exemple une biconvexité, conférant ainsi à la fenêtre (32) un rôle optique.

8.  Interface de couplage selon l'une quelconque des revendications précédentes, dans laquelle l'anneau (3) comporte un col évasé de forme générale tronconique incurvée vers l'extérieur au niveau de son extrémité proximale (311), le col évasé étant apte à être appliqué sur la surface externe du tissu à traiter (2).

9.  Interface de couplage selon l'une quelconque des revendications précédentes, dans laquelle la fenêtre (32) est fixée à l'anneau (3) par collage.

10. Interface de couplage selon l'une quelconque des revendications précédentes, dans laquelle le diamètre intérieur du canal d'irrigation (36) est inférieur au diamètre intérieur du canal d'aspiration (35).

11. Dispositif incluant une source laser et un phacoémulsificateur, le dispositif comprenant un module d'irrigation et un module d'aspiration, **caractérisé en ce que** le dispositif comprend en outre une interface de couplage selon l'une quelconque des revendications précédentes.

12. Dispositif selon la revendication 11, dans lequel le canal d'aspiration de l'interface est connecté au module d'aspiration et le canal d'irrigation est connecté au module d'irrigation.

13. Dispositif selon l'une quelconque des revendications 11 ou 12, lequel comprend un unique module d'irrigation et d'aspiration incluant une pompe et un capteur de dépression, ledit module étant utilisé à la fois pour le traitement laser et le traitement de phacoémulsification.


**Patentansprüche**

1.  Koppelschnittstelle zwischen einer Laserquelle und einem zu behandelnden Gewebe (2), **dadurch gekennzeichnet, dass** die Koppelschnittstelle umfasst:

    - einen Ring (3), der umfasst:

        • eine seitliche Wand (31)
        • ein proximales Ende (311), das dazu bestimmt ist, mit dem zu behandelnden Gewebe (2) in Kontakt zu gelangen, und
        • ein distales Ende (312), das dazu bestimmt ist, das Ende der Laserquelle aufzunehmen,

    - ein Fenster (32), das gegenüber einem von der Laserquelle erzeugten Laserstrahl durchlässig ist, wobei das Fenster auf dichte Weise an dem Ring (3) montiert ist, derart dass das distale Ende (312) des Rings (3) geschlossen ist,
    - einen durchgehenden Saugkanal (35), der sich zwischen der Innen- und der Außenfläche der seitlichen Wand (31) erstreckt, wobei der Saugkanal (35) dazu bestimmt ist, mit einem Saugmodul verbunden zu sein, um die Erzeugung eines Unterdrucks in einem Innenraum (37) zu ermöglichen, der zwischen dem Fenster (32) und der seitlichen Wand (31) definiert ist,
    - einen durchgehenden Irrigationskanal (36), der sich zwischen der Innen- und der Außenfläche der seitlichen Wand (31) erstreckt, wobei der Irrigationskanal (36) dazu bestimmt ist, mit einem Irrigationsmodul verbunden zu sein, um die Infusion von Flüssigkeit (38) in dem Innenraum (37) zu ermöglichen.

2.  Koppelschnittstelle nach Anspruch 1, die ferner eine Blasenfalle umfasst, die sich um das Fenster (32) erstreckt.

3.  Koppelschnittstelle nach Anspruch 2, wobei die Blasenfalle aus einer kreisförmigen Innenrille (33) besteht, die sich zwischen der Seitenwand (31) und dem Fenster (32) erstreckt.

4. Koppelschnittstelle nach einem der vorhergehenden Ansprüche, wobei der Saugkanal (35) sich in einer Ebene erstreckt, die das Fenster (32) enthält.

5. Koppelschnittstelle nach einem der vorhergehenden Ansprüche, wobei das Fenster (32) mit einer Schicht aus wasseranziehendem Material bedeckt ist.

6. Koppelschnittstelle nach einem der vorhergehenden Ansprüche, wobei das Fenster (32) eine nichtebene Geometrie mit parallelen Flächen aufweist, die zum Beispiel eine Konkavität aufweist, die eine bessere Abfuhr der Blasen hin zu der Blasenfalle (312) ermöglicht.

7. Koppelschnittstelle nach einem der vorhergehenden Ansprüche, wobei das Fenster (32) eine nichtebene Geometrie mit nichtparallelen Flächen aufweist, die zum Beispiel eine Bikonvexität aufweist, wodurch dem Fenster (32) eine optische Rolle verliehen wird.

8. Koppelschnittstelle nach einem der vorhergehenden Ansprüche, wobei der Ring (3) einen aufgeweiteten Hals mit allgemein kegelstumpfartiger Form aufweist, der im Bereich seines proximalen Endes (311) nach außen gebogen ist, wobei der aufgeweitete Hals geeignet ist, auf der Außenoberfläche des zu behandelnden Gewebes (2) angebracht zu werden.

9. Koppelschnittstelle nach einem der vorhergehenden Ansprüche, wobei das Fenster (32) durch Kleben an dem Ring (3) befestigt ist.

10. Koppelschnittstelle nach einem der vorhergehenden Ansprüche, wobei der Innendurchmesser des Irrigationskanals (36) kleiner als der Innendurchmesser des Saugkanals (35) ist.

11. Vorrichtung, die eine Laserquelle und einen Phakoemulsifikator umfasst, wobei die Vorrichtung ein Irrigationsmodul und ein Saugmodul umfasst, **dadurch gekennzeichnet, dass** die Vorrichtung ferner eine Koppelschnittstelle nach einem der vorhergehenden Ansprüche umfasst.

12. Vorrichtung nach Anspruch 11, wobei der Saugkanal der Schnittstelle mit dem Saugmodul verbunden ist und der Irrigationskanal mit dem Irrigationsmodul verbunden ist.

13. Vorrichtung nach einem der Ansprüche 11 oder 12, die ferner ein einziges Irrigations- und Saugmodul umfasst, das eine Pumpe und einen Unterdrucksensor umfasst, wobei das Modul zugleich zur Laserbehandlung und zur Phakoemulsifikationsbehandlung verwendet wird.

**Claims**

1. A coupling interface between a laser source and a tissue to be treated (2), **characterized in that** the coupling interface comprises:

    - a ring (3) including:

        • a side wall (31),
        • a proximal end (311) intended to come into contact with the tissue (2) to be treated, and
        • a distal end (312) intended to receive the end of the laser source,

    - a window (32) transparent to a laser beam generated by the laser source, said window being mounted in a sealed manner on the ring (3) so as to close the distal end (312) of the ring (3),
    - a through suction channel (35) extending between the inner and outer faces of the side wall (31), the suction channel (35) being intended to be connected to a suction module to allow the generation of a depression in an internal space (37) defined between the window (32) and the side wall (31),
    - a through irrigation channel (36) extending between the inner and outer faces of the side wall (31), the irrigation channel (36) being intended to be connected to an irrigation module to allow the liquid infusion (38) in the internal space (37) .

2. The coupling interface according to claim 1, which further comprises a bubble trap extending around the window (32) .

3. The coupling interface according to claim 2, wherein the bubble trap consists of a circular inner slot (33) extending between the side wall (31) and the window (32).

4. The coupling interface according to any one of the preceding claims, wherein the suction channel (35) extends in a plane containing the window (32).

5. The coupling interface according to any one of the preceding claims, wherein the window (32) is covered with a layer of hydrophilic material.

6. The coupling interface according to any one of the preceding claims, wherein the window (32) has a non-planar geometry with parallel faces, for example having concavity allowing better discharge of the bubbles towards the bubble trap (312).

7. The coupling interface according to any one of the preceding claims, wherein the window (32) has a non-planar geometry with non-parallel faces, for example having biconvexity, thus giving the window (32) an optical role.

8. The coupling interface according to any one of the preceding claims, wherein the ring (3) includes a flared neck of a generally frustoconical shape curved outwardly at its proximal end (311), the flared neck being able to be applied to the outer surface of the tissue to be treated (2).

9. The coupling interface according to any one of the preceding claims, wherein the window (32) is fixed to the ring (3) by bonding.

10. The coupling interface according to any one of the preceding claims, wherein the internal diameter of the irrigation channel (36) is smaller than the internal diameter of the suction channel (35).

11. A device including a laser source and a phacoemulsifier, the device comprising an irrigation module and a suction module, **characterized in that** the device further comprises a coupling interface according to any one of the preceding claims.

12. The device according to claim 11, wherein the suction channel of the interface is connected to the suction module and the irrigation channel is connected to the irrigation module.

13. The device according to any one of claims 11 or 12, which comprises a single irrigation and suction module including a pump and a vacuum sensor, said module being used both for the laser treatment and the phacoemulsification treatment.

**FIG. 1**

**FIG. 2**

34    A    32    35
312
33
31    36    3
311    37

2    A'    **FIG. 3**

3    1

S = (π * 24 * 4) = 301    S = (π * 24 * 4) - (π * 24 * 2,2) = (24 π * 1,8) = 136

14  2,2
4    1,8
18    18

24    24

2

**FIG. 4A**    **FIG. 4B**

**FIG. 5**

**FIG. 6**

POSITIONNEMENT INTERFACE DE
COUPLAGE SUR OEIL ⟩⟩ 100

ACTIVATION DISPOSITIF
ASPIRATION

ACTIVATION DISPOSITIF
INFUSION

200

300

DESACTIVATION
DISPOSITIF INFUSION ⟩ 400

**FIG. 7**

**EP 3 585 334 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1970034 A

- US 2011319873 A

**Littérature non-brevet citée dans la description**

- **JONATHAN H. TALAMO ; PHILIP GOODING ; DAVID ANGELEY ; WILLIAM W. CULBERTSON ; GEORG SCHUELE ; DANIEL ANDERSEN ; GEORGE MARCELLINO ; EMMA ESSOCK-BURNS ; JUAN BATLLE ; RAFAEL FE-LIZ.** *Optical patient interface in femtosecond laser-assisted cataract surgery: Contact corneal applanation versus liquid immersion*